# EUROPEAN PATENT APPLICATION

(11) **EP 4 141 799 A1**
(43) Date of publication of application: **01.03.2023**
(21) Application number: 22151139.7
(22) Date of filing: 12.01.2022
(51) Int. Cl.: G06T 17/00

(54) **METHOD FOR OBTAINING A CT-LIKE REPRESENTATION AND VIRTUAL X-RAY IMAGES IN ARBITRARY VIEWS FROM A TWO-DIMENSIONAL X-RAY IMAGE**

(30) Priority: 31.08.2021 EP 21194193
(71) Applicant: 1000shapes GmbH, 14167 Berlin (DE)
(72) Inventor: Zachow, Stefan, 14167 Berlin (DE); Lamecker, Hans, 14169 Berlin (DE); Jentsch, Dennis, 12247 Berlin (DE); Ehlke, Moritz, 13357 Berlin (DE)
(74) Representative: Hertin und Partner Rechts- und Patentanwälte PartG mbB

(57) **Abstract**

The invention relates to a method for obtaining a three-dimensional (3-D) representation of a body part of a subject from at least one two-dimensional (2-D) X-ray image of the body part comprising: a) providing at least one 2-D X-ray image of a body part of a subject, b) providing a continuous parametric 3-D model of the body part corresponding to the imaged body part of the subject, c) adjusting at least one feature of the continuous parametric 3-D model to match the corresponding feature of the at least one 2-D X-ray image of the body part of the subject, wherein the at least one feature is (preferably) selected from the group comprising an anatomical landmark, contour, shape, image intensity, structure and silhouette of the at least one 2-D X-ray image of the body part of the subject, thereby generating a 3-D representation of the imaged body part of the subject from the continuous parametric 3-D model, d) generating at least one virtual 2-D X-ray image from the 3-D representation of the imaged body part of the subject generated in step c), e) determining the difference between the at least one virtual 2-D X-ray image and the at least one 2-D X-ray image of the body part of the subject with respect to the at least one feature, f) further adjusting the least one feature of the continuous parametric 3-D model to decrease the difference to the corresponding feature of the at least one 2-D X-ray image of the body part of the subject, thereby generating an improved 3-D representation of the imaged body part of the subject from the continuous parametric 3-D model, g) repeating steps d. to f. until the difference between the at least one virtual 2-D X-ray image generated from the improved 3-D representation and the at least one 2-D X-ray image of the body part of the subject is not further decreased, thereby obtaining a final (or optimized) 3-D representation of the body part of the subject.

## Description

The invention relates to a method for obtaining a three-dimensional (3-D), CT-like, representation and a virtual X-ray image of a body part of a subject from at least one two-dimensional (2-D) X-ray image of the body part comprising: a) providing at least one 2-D X-ray image of a body part of a subject, b) providing a continuous parametric three-dimensional (3-D) model of the body part corresponding to the imaged body part of the subject, c) adjusting at least one feature of the continuous parametric 3-D model to match the corresponding feature of the at least one 2-D X-ray image of the body part of the subject, wherein the at least one feature is (preferably) selected from the group comprising an anatomical landmark, contour, shape, image intensity, structure and silhouette of the at least one 2-D X-ray image of the body part of the subject, thereby generating a 3-D representation of the imaged body part of the subject from the continuous parametric 3-D model, d) generating at least one virtual 2-D X-ray image from the 3-D representation of the imaged body part of the subject generated in step c), e) determining the difference between the at least one virtual 2-D X-ray image and the at least one 2-D X-ray image of the body part of the subject with respect to the at least one feature, f) further adjusting the least one feature of the continuous parametric 3-D model to decrease the difference to the corresponding feature of the at least one 2-D X-ray image of the body part of the subject, thereby generating an improved 3-D representation of the imaged body part of the subject from the continuous parametric 3-D model, g) repeating steps d. to f. until the difference between the at least one virtual 2-D X-ray image generated from the improved 3-D representation and the at least one 2-D X-ray image of the body part of the subject is not further decreased, thereby obtaining a final (or optimized) 3-D representation of the body part of the subject.

In a particular embodiment the invention relates to a method further comprising h) generating a virtual computed tomography (CT) data set from the final 3-D representation of step g), wherein the continuous parametric 3-D model not only comprises data on variations in shape but also in image intensity learned from CT-data sets from which the continuous parametric 3-D model is derived from, wherein the at least one adjusted feature comprises image intensity, and wherein step h) comprises sampling the intensity data of the final 3-D representation to a regular volumetric grid (3-D scalar or vector field), thereby modelling the intensity data in a volume inside and/or outside of the final 3-D representation.

The invention further relates to a method for i) generating at least one virtual 2-D X-ray image from the virtual CT data set generated in step h), wherein step i) comprises defining a virtual X-ray setup comprising at least a source and a detector, virtually arranging the virtual CT data set between the source and the detector of the virtual X-ray setup, and simulating the X-ray process within the virtual X-ray setup to create at least one virtual 2-D X-ray from the virtual CT data set. The invention further relates to a device or system comprising means for carrying out the steps of the method according to the invention, a computer program [product] comprising instructions which, when the program is executed by a computer, cause the computer to execute the steps of the method according to the invention and a computer-readable storage medium having stored thereon said computer program.

Among others, the invention relates to the use of a 3-D representation obtained according to the method of the invention, for the construction of a joint prosthesis or an osteosynthesis plate or prosthesis or for the planning and/or the evaluation of the outcome of a surgical procedure and many other applications including diagnosis not only of the body part itself but also relative spatial proportions or adjacent anatomical structures.

### BACKGROUND OF THE INVENTION

Conventional projective 2-D radiographs (2-D X-rays) are the gold standard for analyzing bony structures since they show bone and surrounding soft tissue at high contrast and resolution. However, each X-ray projection reduces 3-D information to a 2-D projection image in which all structures in the direction of the X-ray beams are shown superimposed (summation). The exact 3-D shape and density distribution of the imaged object(s) are not directly evident from the resulting 2-D image, as said 2-D image provides few information how the imaged 2-D object was oriented in the beam path and how partial structures (e.g. joint socket) are spatially oriented. However, this information is extremely important for medical diagnosis and treatment planning. Metric information cannot easily be determined from a 2-D X-ray image either, as the perspective projection leads to scaling effects. Objects that are close to the radiation source appear larger in the image than objects that are further away from the source.

As an alternative to planar 2-D X-rays, tomographic recording methods, such as computed tomography (CT) or magnetic resonance imaging (MRI), are often used. These provide the exact representation of the respective anatomy in a metrically correct and three-dimensional manner. However, X-ray tomography methods are more expensive than planar X-rays, more time-consuming to create the image data, lead to a higher radiation dose, are not always and everywhere available and, moreover, these recordings are always made in a lying position, which means that an assessment of the joint situation in an upright standing position under load by the body weight is not possible or is only possible to a limited extent.

Hence, despite the advantages of 3-D image acquisition methods, 2-D X-ray imaging is still widely used for medical diagnosis, treatment planning, and follow-up, e.g. in the context of orthopedics or trauma and reconstructive surgery. Compared to 3-D computed tomography (CT), 2-D radiography exposes the patient to less radiation, is widely available, inexpensive, and enables fast and flexible screening in the operating room via mobile C-arms. Further, 2-D X-ray is required for imaging of weight-bearing situations and applied for dynamic imaging of joint motion (fluoroscopy), neither of which can be performed with tomographic imaging such as CT or MRI. Today, implants of appropriate type and size for joint-replacement surgery are still chosen by manual overlaying 2-D templates of the implant's silhouette over an anterior-posterior (AP) radiograph that shows the respective body part. As 2-D radiographic measurements are limited to the X-ray plane only, measurements can be interpreted wrongly if the target anatomy is rotated out-of-plane, does otherwise not coincide with some expected pose or scale, or if a separation between structures of interest and background structures remains unclear.

These ambiguities have been hard to resolve so far and the outcome of X-ray-based assessment largely depends on the radiologists and surgeons to cope with uncertainties in the given images. Radiographic assessments have hence been associated with low inter-observer reliability in a number of studies (Aydin et al., 2017; Clohisy et al., 2009). Naturally occurring variations in pelvic tilt, for example, cause non-uniform magnification patterns in AP radiographs due to out-of-plane alignment of the hip with the X-ray detector. Any disregard of this tilt can lead to malpositioning of the cup-component and thus dislocation after surgery. Other examples are unknown leg flexion in AP radiographs for the assessment of the knee joint, or lateral malformation of the spine as in scoliosis in lateral spinal radiographic imaging. Since measurements from conventional 2-D radiographs do not have the required accuracy or reliability, surgeons often either rely on their experience or on additional preoperative CT scans for obtaining the patient-specific 3-D information, which in the latter case comes at a higher radiation dose and does not account for joint arrangements under load.

Assessing anatomical parameters accurately in 3-D becomes increasingly important with the availability of surgical drilling or fixation guides, patient specific customized instrumentation, and intraoperative navigation systems such as robots, as well as for per-operative planning and post-operative assessment.

Thus, there is a need for technologies that provide such 3-D information without the burden of additional or elevated radiation exposure and costs.

Methods for reconstructing 3-D anatomical shape from a limited number of 2-D radiographs have been proposed for a wide range of clinical applications, including hip replacement planning (Zheng, 2009; Schumann et al., 2015) and pose estimation of the knee from biplanar imaging to compute knee kinematics (Baka et al., 2011, 2014). A common concept of many 3-D reconstruction approaches is to search among variations of anatomical 3-D representations, also referred to as candidate representations, until a representation is found that best matches the information depicted in the given 2-D radiographs. The best fit is then assumed to also be the best approximation of the true 3-D anatomy depicted in the X-rays.

While the field of reconstructing 3-D bone from 2-D X-rays has been well studied in recent years, the proposed approaches often share one or several practical limitations. For example, feature extraction such as extracting contours in the X-rays required for silhouette-based reconstruction is a challenging task and is in general performed manually (Dworzak et al., 2010) or semiautomatically (Lamecker et al., 2006; Zheng, 2009).

A known problem of the approach to reconstruct a single-bone is that 2-D radiographs typically depict multiple bony structures and a missing a-priori knowledge about the possible joint arrangements can result in inconsistencies such as overlaps and out-of-plane errors.

Some proposed dual X-ray reconstruction methods require two calibrated X-ray images from different views as an input (Whitmarsh et al., 2011; Baka et al., 2014; Karade and Ravi, 2015). In a clinical setting, however, there might only be one X-ray available at a time or the exact relative calibration of multiple X-rays required for a faithful 3-D reconstruction might be unknown.

Another approach proposes the use of digitally reconstructed radiographs (DRR). Most existing methods for the generation of DRRs are tailored for rigid registration, where only the transformation and translation of an anatomical model are changed iteratively in the matching procedure. Non-rigid, intensity-based reconstruction, however, requires the computation of additional updates to the geometry and, depending on the approach, to the radiodensity distribution expressed by the model. The efficient generation of DRRs from deformable (articulated) anatomical models is crucial for overall 2-D/3-D reconstruction speed but hasn't been addressed to a satisfactory level in the context of non-rigid 2-D/3-D reconstruction frameworks.

In light of the prior art there remains a significant need in the art to provide additional means for the precise, reliable, fast and reproducible reconstruction of objects, such as body parts or bones, from 2-D radiographs to 3-D models of said objects.

Furthermore, there is a need to integrate this additional 3-D information into the existing (planning) tools in a user-friendly way in order to enable a widespread adoption.

### SUMMARY OF THE INVENTION

The above-mentioned problem is solved by the features of the independent claims. Preferred embodiments of the present invention are provided by the dependent claims.

The solution proposed with the present invention generates virtual X-ray images that imitate clinical radiographs through projection of (articulated) statistical shape and intensity models (SSIMs and A-SSIMs). For this purpose, the X-ray source and detector of a real X-ray setup are mapped to a virtual setup, in which the subject-specific anatomy is represented by a tetrahedral mesh representing the anatomical shape together with the associated radiodensity information. Back-projected anatomical landmarks are utilized to robustly position the A-SSIM in the virtual 3-D setup according to the real X-ray setup and to derive initial shape and intensity candidates. A virtual X-ray generator (source) acts as a point source that casts X-ray beams through the mesh towards an image plane (detector). The direction and length of the beams are obtained via a camera model, where the camera points from the source towards the detector and has a focal length equal to the distance between X-ray source and detector/image plane (SDD, SID). Following an idealized physical model of X-ray attenuation, each pixel in the image plane is assigned the intensity of an associated beam after accumulating its respective attenuation on its way through the tetrahedral mesh representation. Virtual X-ray images, hence, are the result of a volumetric, perspective projection that is characterized by a perspective projection matrix.

The invention therefore relates to a method for obtaining a three-dimensional (3-D) representation of a body part of a subject from at least one two-dimensional (2-D) X-ray image of the body part comprising a) providing at least one 2-D X-ray image of a body part of a subject, b) providing a continuous parametric 3-D model of the body part corresponding to the imaged body part of the subject, c) adjusting at least one feature of the continuous parametric 3-D model to match the corresponding feature of the at least one 2-D X-ray image of the body part of the subject, wherein the at least one feature is (preferably) selected from the group comprising an anatomical landmark, contour, shape, image intensity, structure and silhouette of the at least one 2-D X-ray image of the body part of the subject, thereby generating a 3-D representation of the imaged body part of the subject from the continuous parametric 3-D model, d) generating at least one virtual 2-D X-ray image from the 3-D representation of the imaged body part of the subject generated in step c), e) determining the difference between the at least one virtual 2-D X-ray image and the at least one 2-D X-ray image of the body part of the subject with respect to the at least one feature, f) further adjusting the least one feature of the continuous parametric 3-D model to decrease the difference to the corresponding feature of the at least one 2-D X-ray image of the body part of the subject, thereby generating an improved 3-D representation of the imaged body part of the subject from the continuous parametric 3-D model, and g) repeating steps d. to f. until the difference between the at least one virtual 2-D X-ray image generated from the improved 3-D representation and the at least one 2-D X-ray image of the body part of the subject is not further decreased, thereby obtaining a final (or optimized) 3-D representation of the body part of the subject.

An aim of the method according to the invention is to find the model instance out of the A-SSIMs that matches the depiction in the given 2-D radiograph best, as it is assumed to represent a good approximation of the true, subject-specific anatomy. This is achieved by means of an optimization process, in which virtual X-ray images that mimic real X-rays of the anatomical structures of interest are generated from at least one or more 3-D mesh variations until the similarity between the projective depiction of the respective anatomical structures, i.e. the virtual X-ray images and the given clinical images is maximized.

The proposed 2-D/3-D reconstruction framework registers A-SSIMs to the at least one X-ray with the benefits of (1) an enhanced prior due to the combined statistical analysis over the shape and radiodensity of all involved structures; (2) increased robustness since multiple, possibly articulating bony structures are modeled together and projected simultaneously; and (3) a consistent joint posture as the relative transformation of individual structures of the joint is subject to a prior (i.e. lack of out-of-plane errors between anatomies).

The generation of virtual X-ray images is computationally expensive, as many virtual X-ray beams (e.g. one for every pixel) have to be emulated to project the anatomy of interest. To compute virtual X-rays quickly, the method proposed herein relies on parallel execution on Graphical Processing Units (GPUs), combining the idea of cell-based ray casting (Georgii and Westermann, 2006; Weiler et al., 2003) with ray-tetrahedron intersection tests. The approach does not require the classification of the projected tetrahedral shape for tessellation from previous work (Sadowsky et al., 2006, 2005) and can hence be implemented efficiently in GPU shader programs without any explicit branching or looping. In addition, a technique is proposed to combine both the deformation of (A-)SSIMs and their projection on the GPU in order to avoid copy operations between main memory and GPU memory, and to increase the deformation and projection throughput further.

The rendering speed of the herein proposed method exceeds those of the state of the art projected tetrahedra method when projecting tetrahedral meshes with more than 300k cells. This is due to a reduced number of per-geometry operations and the lack of branching and looping operations compared to other approaches. The time for deforming tetrahedral meshes on a CPU, as proposed by prior art methods, grows linearly with the number of linear combinations between deformation parameters and additional transformation parameters (shift, scale, articulation, etc.) of the SSIM. The method according to the invention benefits from parallel execution of the linear combination and vector operations on the GPU. The method proposed herein provides means for fast and efficient generation of virtual X-ray images from SSIMs and A-SSIMs, wherein SSIMs are the foundation for generating plausible instances of anatomical shape and radiodensity through deformation. This implementation takes the deformation and articulation of A-SSIMs into account, shifting the computational burden from the CPU to the GPU to make the projection of virtual X-rays applicable for matching 3-D models to 2-D radiographs. In context of intensity-based 2D/3D reconstruction, the GPU-only approach has noteworthy benefits over previous prior art CPU-based and hybrid CPU/GPU methods:
First, the combined deformation and projection on the GPU shows a performance increase in terms of rendering speed. Faster rendering speeds directly translate to speed-ups generating virtual X-ray images and thus matching SSIMs or A-SSIMs to clinical X-rays. Second, the GPU-only method scales better with respect to the number of deformation parameters and the resolution (e.g. number of tetrahedra) of the underlying model. This allows for projecting anatomical models that are more accurate in representing detailed individual anatomy. Third, virtual X-ray images are rendered directly to GPU texture memory and are hence available for further processing on the GPU. The reconstruction framework proposed herein, for instance, only reads back the final similarity value (one scalar per image) to CPU memory, benefiting from full GPU parallelization during similarity evaluation while avoiding costly back-and-forth copy operations of images.

It was therefore entirely surprising that compared to previous approaches for generating virtual X-rays from SSIMs and A-SSIMs, the proposed method achieves comparable quality at much higher framerates, making this approach particularly useful for non-rigid 2-D/3-D registration tasks that have not yet found their way into clinical applications due to the high computing power required and the associated high time expenditure.

In one embodiment the invention relates to a method for obtaining a three-dimensional (3-D) representation of a body part of a subject from at least one two-dimensional (2-D) X-ray image of the body part, wherein at least one X-ray marker preferably of defined dimension is present in the 2-D X-ray image and wherein the at least one X-ray marker is used to determine at least one feature selected from the group comprising an anatomical landmark, the distance between the imaged body part and the X-ray source and/or the detector, the size and/or orientation of the imaged body part.

The precision of the present method in generating 3-D models of a body part from 2-D X-ray images can be further improved, if certain distance measurements and/or empty measurements of the following configurations are carried out and this information is integrated directly into the evaluation parameters (DICOM metadata): a) distance between X-ray source and detector, b) measurement of the distance between X-ray source and the front of the subject, c) measurement of the distance between the detector and the rear of the subject and d) measurement of the distance between the detector and a defined landmark of the subject. Upon integration of this data into the generation of the 3-D representation according to the method of the invention the precision and reproducibility of generating 3-D models from 2-D X-ray images can be improved significantly.

The proposed reconstruction method can be applied, in one embodiment, in the context of clinical applications in hip-joint replacement and osteosynthesis, wherein said applications benefit from the invention for treatment planning and postoperative follow-up in orthopedics or reconstructive surgery. Other conceivable applications are knee, shoulder, or spine surgery.

First, ambiguities in the X-rays are resolved by the method of the present invention in a reproducible fashion, addressing the issue of inter-observer reliability. Second, anatomical structures can be visualized from arbitrary viewpoints in 3-D space. Third, 3-D parameters of interest that were previously not accessible through 2-D templating, such as the pelvic tilt or the orientation of the acetabulum or leg flexion/extension or spinal curvature, can be measured directly on the reconstructed 3-D anatomies. Finally, the reconstructed anatomical model can be further processed, among others, to create a patient-specific therapeutic plan by predicting the load on a planned implant through functional analysis in 3-D space from fluoroscopy or for diagnosis or post-operative 3-D assessment of the outcome.

In another preferred embodiment the invention relates to a method for obtaining a three-dimensional (3-D) representation of a body part of a subject from at least one two-dimensional (2-D) X-ray image of the body part, further comprising step h. generating a virtual computed tomography (CT) data set from the final 3-D representation of step g.

This unexpected effect of the invention is in embodiments achieved by combining multiple features. First, the continuous parametric 3-D model is extended by data on variations in image intensity from CT-data sets associated with the continuous parametric 3-D model. Second, the intensity data of the final 3-D representation needs to be sampled to a regular volumetric grid (a 3-D scalar or vector field) and thereby modelling the intensity data in a volume inside and/or outside of the final 3-D representation. And third, the grid, containing the intensity data, is sampled to a CT-like dataset.

Accordingly, in said preferred embodiment CT-like datasets ("pseudo-CT") of the final/optimized 3D representation can be generated from the final articulated 3-D statistical shape and intensity model (A-SSIM).

These virtual or "pseudo CTs" of the respective body part allow to perform any 3-D analysis as if one is working with a regular 3-D CT, such as with existing software systems that process 3-D DICOM data.

One of the additional technical effects of the technical features of this preferred embodiment, is that to a radiologist or surgeon familiar visualizations, namely a CT dataset, are created that can be used in existing software solutions (e.g. for surgery or implant planning, and navigation during surgery). The features of the present invention that achieve this surprising effect are not obvious to the skilled person from prior art techniques, as they require additional procedural steps, which are not obvious from said prior art and result from years of research by the inventors. The method of the present invention also differs from other work, which generally applies machine learning approaches to learn CT data from 2-D X-rays to estimate 3-D representations of x-rayed body parts. This approach differs substantially from the present method.

It is also possible to generate from the virtual (pseudo) CT virtual 2-D X-rays (DRRs) in arbitrary projections. These virtual 2-D X-rays can help to normalize or correct the original (real) 2-D X-ray image to display relevant anatomical aspects in standardized views, for example, using 2-D image viewers in existing medical image software systems. These virtual 2-D X-rays can also help in positioning patients in the operating room (OR) according to a planned position, as for instance in radiotherapy.

In one embodiment of the method according to the invention the continuous parametric 3-D model comprises data on variations in image intensity from CT-data sets associated with the continuous parametric 3-D model, wherein the at least one adjusted feature comprises image intensity, and wherein step h. comprises sampling the intensity data of the final 3-D representation to a grid, thereby modelling the intensity data in a volume inside and/or outside of the final 3-D representation.

In some embodiments the continuous parametric 3-D model comprises data on variations in shape and image intensity learned from CT-data sets [from which] the continuous parametric 3-D model is derived from.

In some embodiments the grid is a regular volumetric grid. In some embodiments the grid is a 3-D scalar or vector field. Accordingly, in some embodiments of the method according to the invention the continuous parametric 3-D model comprises data on variations in shape and image intensity learned from CT-data sets the continuous parametric 3-D model is derived from, wherein the at least one adjusted feature comprises image intensity, and wherein step h. comprises sampling the intensity data of the final 3-D representation to a regular volumetric grid, thereby modelling the intensity data in a volume inside and/or outside of the final 3-D representation.

In one embodiment the invention relates to a method for obtaining a three-dimensional (3-D) representation of a body part of a subject from at least one two-dimensional (2-D) X-ray image of the respective body part, wherein at least one X-ray marker preferably of defined dimension is present in the 2-D X-ray image and wherein the at least one X-ray marker is used to determine at least one feature selected from the group comprising an anatomical landmark, the distance between the imaged body part and the X-ray source and/or the detector, the size and/or orientation of the imaged body part.

In one embodiment the invention relates to a method for obtaining a three-dimensional (3-D) representation of a body part of a subject from at least one two-dimensional (2-D) X-ray image of the body part, further comprising i) generating at least one virtual 2-D X-ray image from the virtual CT data set generated in step h).

In one embodiment the invention relates to a method for obtaining a three-dimensional (3-D) representation of a body part of a subject from at least one two-dimensional (2-D) X-ray image of the body part, wherein step i) comprises: defining a virtual X-ray setup comprising at least a source and a detector, virtually arranging the virtual CT data set between the source and the detector of the virtual X-ray setup and simulating the X-ray process within the virtual X-ray setup to create at least one virtual 2-D X-ray from the virtual CT data set.

In embodiments the invention further relates to a method for i) generating at least one virtual 2-D X-ray image from the virtual CT data set generated in step h), wherein step i) comprises defining a virtual X-ray setup comprising at least a source and a detector, virtually arranging the virtual CT data set between the source and the detector of the virtual X-ray setup, and simulating the X-ray process within the virtual X-ray setup to create at least one virtual 2-D X-ray from the virtual CT data set.

In one embodiment of the method according to the invention the imaged body part is a severed body part and wherein the continuous parametric 3-D model and/or the (final) 3-D representation are a healthy or intact representation of the severed body part. In some embodiments the continuous parametric 3-D model and/or the (final) 3-D representation are a healthy, intact or native representation of the severed body part.

In one embodiment of the method according to the invention the imaged body part is a severed or pathological body part and the continuous parametric 3-D model and/or the (final) 3-D representation are a healthy, intact or native representation of the severed or pathological body part. In one embodiment of the method according to the invention the imaged body part is a pathological body part and the continuous parametric 3-D model and/or the (final) 3-D representation are a healthy, intact or native representation of the pathological body part.

In one embodiment the invention relates to a method for obtaining a three-dimensional (3-D) representation of a body part of a subject from at least one two-dimensional (2-D) X-ray image of the body part, wherein a continuous fluoroscopic 2-D X-ray image sequence (video-radiography) of a body part in motion or articulation of a subject is provided, and wherein the 3-D representation for each image of the sequence yields a moving or articulating kinematic 3-D representation of the respective body part that is imaged in motion.

In embodiments of the invention a continuous fluoroscopic (real-time moving) 2-D X-ray image of a moving body part of a subject is provided, and wherein the (final) 3-D representation is a moving 3-D representation of the imaged body part in motion.

In one embodiment of the method according to the invention the body part of the subject is selected from the group of one or more organ(s), limb(s), bone(s), joint(s) and/or a portion and/or a combination thereof.

In one embodiment of the method according to the invention the body part of the subject comprises at least one implant, screw, plate, joint prosthesis, osteosynthesis plate and/or any combination thereof.

The invention further relates to a device or system comprising means for carrying out the steps of the method according to the invention, and preferably a graphical user interface system configured to display the (final) 3-D representation and/or the at least one virtual CT data set, generated according to the method of the invention, of the body part of the subject.

The invention further relates to a computer-program [product] for obtaining a three-dimensional (3-D) representation of a body part of a subject from at least one two-dimensional (2-D) X-ray image of the body part of the subject, comprising instructions which, when the program is executed by a computer, cause the computer to execute the method steps of the method according to the invention and to display the (final) 3-D representation and/or the at least one virtual CT data set, generated according to the method of the invention, of the body part of the subject on a graphical user interface system.

In embodiments of the invention the computer-program [product], comprises instructions which, when the program is executed by a computer, cause the computer to execute the method steps of the method according to the invention and to yield the (final) 3-D representation and/or the at least one virtual CT data set, generated according to the method of the invention, of the body part of the subject for either the display on a graphical user interface system or in any 3-D printable file format such as STL.

The invention further relates to a computer-readable storage medium having stored thereon the computer program according to the present invention.

The invention further relates to the use of a 3-D representation, relating to the shape and appearance of at a body part, and obtained according to the method of the invention for the construction of a joint prosthesis or an osteosynthesis plate or prosthesis.

In embodiments of the invention the use of a 3-D representation, relating to the shape and appearance of at a body part, and obtained according to the method of the invention, for the configuration of a joint prosthesis or an osteosynthesis plate or a prosthesis or customized instrumentations such as surgical drill or cutting guides.

The invention further relates to the use of a 3-D representation obtained according to the method of the invention, for the planning and/or the evaluation of the outcome of a surgical procedure on the imaged body part.

In embodiments the invention relates to the use of a 3-D representation obtained according to the method of the invention, for the planning or the execution of an intervention and/or for the evaluation of the outcome of a surgical procedure on the imaged body part.

Each feature of the invention that is disclosed in the context of one aspect of the invention is herewith also disclosed in the context of the other inventive aspects disclosed herein. Accordingly, embodiments and features of the invention described with respect to the method disclosed herein, are considered to be disclosed with respect to each and every other aspect of the disclosure, such that features characterizing one embodiment of the present method, may be employed to characterize another embodiment of the method or it's use, the computer program product or device and vice-versa. The various aspects of the invention are unified by, benefit from, are based on and/or are linked by the common and surprising finding of the unexpected advantageous effects of the present method in generating a 3-D representation or virtual CT of a body part from at least one 2-D image.

### DETAILED DESCRIPTION OF THE INVENTION

In the context of the present invention the term **"three-dimensional (3-D) representation"** refers to a virtual or *in silico* three-dimensional representation or model of an object, such as a body part, that has a maximum similarity or provides the closest representation of the real imaged object, e.g. body part, that can be generated according to the method of the present invention. Said representation shares one or more distinctive and characteristic features with the imaged object and can serve as a digital twin or virtual representation of said object.

In the context of the present invention a body part of a subject may comprises in one embodiment at least one implant, screw, plate, joint prosthesis, osteosynthesis plate and/or any combination thereof.

In one embodiment of the present invention an implant, screw, plate, joint prosthesis and/or osteosynthesis plate may be comprised of one or more materials comprising, for example, metal, plastics, material based on animal or human bone or cartilage, a human bone, an animal bone, human cells, animal cells, glass fiber, titanium, carbon, stainless steel, wood, rubber, aluminum, polyethylene, polypropylene, acrylics, ceramics, polyurethane and /or composite materials, such as carbon fiber reinforced polymers or any combination thereof.

Herein the term " **body part"** refers to a whole body, a part or portion of a body, one or more organ(s), limb(s), bone(s), joint(s)), relative spatial proportions or adjacent anatomical structures and/or a portion and/or a combination thereof. It may also comprise of one embodiment of at least one implant, screw, plate, joint prosthesis, osteosynthesis plate and/or any combination thereof.

In the context of the present invention the term " **subject"** refers to an individual, a patient, a human, an animal, a representation or avatar of a human or an animal, a prosthesis.

The term **"two-dimensional (2-D) X-ray image"** refers to an X-ray image generated or provided by casting X-rays through a body part of a subject positioned between an X-ray source and a detector, wherein X-ray herein refers to any form of high-energy electromagnetic radiation or Röntgen radiation. An X-ray image may be generated by X-rays passing through the imaged body part and reaching an X-ray detector behind the imaged body part, wherein an image will be formed that represents the perspective radio-opacity projection formed by the body part itself and all objects inside the body part. Herein a type of X-ray detector may be photographic film, or any other type of detector capable of generating digital images. Herein, 2-D X-ray images resulting from an X-ray process may be also called radiographs or 2-D radiographs.

Usually, a radiograph is acquired according to a physical X-ray acquisition setup (an X-ray acquisition device or system), in which the position of the X-ray source relative to the detector plate (i.e. the X-ray film in analogue systems) and the size of the detector determine the perspective of the imaged subject or body part onto the 2-D radiograph. Depending on the setup, degrees of freedom in X-ray acquisition also include the distance between the X-ray source and the imaged subject/object or body part (SSD, SOD), the distance between imaged subject/object or body part and the detector/image plane (ODD, OID), the field-of-view angles (opening angles) if they are artificially restricted through collimators, and the relative positioning of X-ray equipment when multiple 2-D images are taken.

Herein **"2-D"** refers to two-dimensional or bi-dimensional, which is a geometric setting in which two parameters are required to determine the position of an element in a 2-D domain, wherein a 2-D image may represent a planar image. Accordingly, "3-D" refers herein to three-dimensional, which is a geometric setting in which three parameters are required to determine the position of an element in a 3-D domain. Herein "3-D modeling" may refer to a process of developing a mathematical coordinate-based representation of a surface as well as the interior of an object, for example an imaged body part, in three dimensions.

In the context of the present invention the term **"continuous parametric 3-D model"** refers to a 3-D model, which is deformable, wherein the deformation is controlled via a finite set of continuous parameters. The continuous parametric 3-D model in the context of the invention also carries a set of fields, e.g. scalar, vector, or tensor valued quantities, defined on the 3-D model domain, which are also parameterized continuously by another finite set of parameters. The way the parameters actually deform the 3-D model or change its fields, is derived from training data that represents a respective distribution within a population of individual distinct representations of such 3-D models. In this way, the continuous parametric 3-D model is capable of synthesizing an infinite number of yet unseen such representations by interpolation within the respective parameter space.

In scientific or medical visualization volume rendering or volume ray-casting comprise a set of techniques used to display a 2-D projection of a 3-D discretely sampled data set, typically a 3-D scalar field as obtained via tomographic imaging. As an example, a 3-D data set can be a consecutive sequence of 2-D cross-sectional images acquired e.g. by an MRI or CT scanner. Cross-sectional images can be either acquired in a regular pattern (i.e, a specific uniform distance) or in an irregular pattern (i.e., with varying distances). Each cross-sectional image of a tomographic scan has the same dimensions (i.e., the same extent in horizontal or vertical direction, namely the image width and image height). Cross-sectional images of a tomographic scan are represented by a discrete and equal number of rectangular picture elements (pixels), where picture elements have two, not necessarily equal dimensions (pixel width and pixel height) that remain constant for all cross-sectional images of a particular tomographic scan. In a tomographic measurement, cross-sectional images are taken at particular locations of a scanned subject, where image pixels do represent spatial measurements at a pixel-centered location. Stacking all cross-sectional images according to their spatial locations results in a 3-D scalar field consisting of regularly distributed volume elements (voxels) having the pixel dimensions within the cross-sectional plane and the distance of its adjacent cross-sectional images as a third dimension. The discrete digital representation of stacked cross-sectional images is an example of a "regular volumetric grid", with each volume element, or "voxel" represented by a single value that is obtained by sampling the immediate area surrounding that voxel.

Herein the term **"digitally reconstructed radiograph (DRR)"** refers to a method for emulating the X-ray attenuation based on CT-data to generate a 2-D image.

**"Statistical shape models (SSM)"** herein refers to an extension of the point distribution models with intensity information as proposed by Yao (2002). They are generated by computing a principal component analysis (PCA) from training sets of tetrahedral meshes with associated radiodensity information from CT. Each training mesh represents a subject-specific anatomy according to the tetrahedral representation with Bernstein polynomials, according to the present invention. Analogous to SSMs, **Statistical shape and intensity models (SSIM)** combine the mean shape and intensity distributions of all training data and their specific variations, expressed in the PCA eigenvectors. Further extending the concept of SSIMs, **articulated statistical shape and intensity models (A-SSIM)** are proposed here. A-SSIMs capture the statistical variation of joint structures in terms of bone shape and radiodensity across a training population from CT and express the relative posture of individual joint components.

The term **"feature"** refers to an anatomical landmark, contour, shape, image intensity, radiodensity, bone mineral density, structure and silhouette of the at least one 2-D X-ray image of the body part of the subject, of the imaged body part of the subject itself, of the at least one virtual 2-D X-ray image of the body part and/or of the 3-D model or representation of the imaged body part. In one embodiment, a feature that is compared, matched and/or adjusted in the virtual X-ray image, and/ or the 3-D representation or model to match or mimic the corresponding feature of the imaged body part is present in the real and the virtual 2-D X-ray image, as well as in the 3-D representation or model of the imaged body part and/or the imaged body part itself.

The term **"anatomical landmark"** refers to a distinguishing spatial marker, spot or region in the imaged anatomy of the body part which can be determined in the at least 2-D Xray image of the body part, wherein the corresponding spatial marker, spot or region is determined in the continuous parametric 3-D model, the virtual 2-D X-ray image and the 3-D representation and model of the imaged object. Herein, anatomical landmarks are 2-D landmarks derived from the clinical/"real" 2-D X-ray images. The derived landmarks are then applied to initialize the A-SSIM for shape and intensity-based reconstruction.

Herein the term **"shape"** refers to a form, format, pattern, outline, silhouette, embodiment, architecture or configuration of an object, such as a body part or structures comprised within said body part.

Herein the term **"image intensity"** refers to perceived appearance, image intensity information, information on the intensity of an X-ray imaged body part, bone density information depicted in an X-ray, intensity information based on radio-densities or radio-opacities.

**"Image registration"** refers to a process of transforming different sets of data into one coordinate system, wherein the data may be multiple images or radiographs. Intensity-based automatic image registration is an iterative process based on a pair of images. Image registration involves spatially transforming the source/continuous images (continuous parametric 3-D model) to align with the target image (2-D X-ray of a body part). The reference frame in the target image is stationary, while the other datasets are transformed to match to the target. Intensity-based methods compare intensity distribution patterns in images via correlation metrics.

In the context of the present invention the term **"structure"** refers to texture, morphology, formation, makeup, subdivision, formation of or comprised within an object, such as a body part.

Herein the term **"silhouette"** refers to an outline, contour, profile, adumbration or inner and outer border of an object, such as a body part, or any physical structure comprised within a body part.

In the context of the present invention the term **"virtual 2-D X-ray"** refers to an *in silico* generated image mimicking or representing a real 2-D X-ray image. Herein a virtual 2-D Xray image may be generated according to the method of the present invention from a 3-D representation or model of a body part and/or from a virtual ("pseudo") or a real CT dataset of a body part.

Herein the term **"difference"** between a virtual 2-D X-ray image and a real 2-D X-ray image may be a difference, distinction or divergence between any feature of a virtual or real 2-D X-ray image according to the present invention. The unit or manner of the difference, distinction or divergence depends on the feature it refers to as well as the chosen metric the similarity measure is evaluated with.

In the context of the present invention the term **"improved 3-D representation"** refers to a 3-D representation or 3-D model whose virtual X-ray projection was optimized, adjusted and/or modified to better fit, mimic or resemble the object or body part it is supposed to approximate within a real X-ray image. In one embodiment such improvement or adjustment is achieved by adjustment or modification of at least one feature present in the 3-D representation and the corresponding object or body part or a 2-D X-ray image of said body part.

In the context of the present invention the term **"final (or optimized) 3-D representation of the body part of the subject"** refers to a 3-D model or 3-D representation that comprises the least divergence or difference in all compared features, which cannot be further decreased or minimized according to the method of the present invention. In other words final (or optimized) 3-D representation of the body part of the subject represents the 3-D model or representation that represents or mimics the imaged body part the most and comprises an overlap or similarity to the body part that cannot be increased or optimized further by the method according to the present invention.

In the context of the present invention the term **"X-ray marker"** refers to anatomical or fiducial markers, also known as: anatomical side markers, Pb markers, lead markers, x-ray lead markers, or radiographic film identification markers, that are used to mark x-ray films. They are used on radiographic images to determine anatomical side of body, date of the procedure, and may include, e.g., a subject's name. X-ray markers may be used to indicate positioning of the body e.g. prone or supine, left or write, or as to time when performing procedures. X-ray markers may be objects, such as letters, numbers or shapes of defined dimensions made of lead or any other metal or metal ligation. Herein an X-ray marker may be defined in its dimension, namely its size, height, width, thickness, form and/or material or material composition. One or more X-ray markers may, in one embodiment of the invention, be used to determine or estimate the size of an imaged body part or individual and/or, the distance between the source and/or detector and the imaged body part or individual. In another or the same embodiment of the invention one or more X-ray markers may be used to determine or estimate the location of an anatomical landmark of the imaged body part or individual with respect to the same or a different body part or with respect to the entire body of the imaged individual. In one embodiment of the invention one or more X-ray markers may be used that are identical or different in their dimensions.

X-ray markers are particularly useful, as magnification effects do occur due to the perspective geometry of X-ray projections and can be standardized across patients by adhering to imaging protocols, where each subject or body part is imaged in a well-defined position between radiation source and detector and/or by attaching X-ray/radiopaque markers of known shape and size to the subject or body part during X-ray acquisition and relating to their projected size in the at least one X-ray image.

In the context of the present invention the term **"virtual computed tomography (CT) data set"** or **"pseudo-CT"** refers to a data set comprising image information of a 3-D model of a body part generated from at least one 2-D X-ray image according to the method of the present invention, wherein said image information is displayed mimicking a real CT-dataset of that particular anatomical structure (body part). Said virtual or pseudo-CT dataset may, in one example, depict cross sections or "slices" throughout the X-ray-imaged body part. Said slices or cross sections show image information of said 3-D model of the imaged body part separated by a defined spatial distance, e.g. a cross section every 1, or every X, millimeters from top to bottom of said 3-D model of a body part, wherein X may be any desired spatial distance.

Herein a **"a virtual X-ray setup"** refers to an *in silico* existing X-ray setup mimicking or representing a real functional X-ray imaging setup, wherein said virtual X-ray setup is configured to perform or mimic any function a real X-ray imaging setup may perform. The virtual X-ray setup according to the invention comprises of one embodiment at least a "source" and "a detector", wherein the source and the detector are virtual and configured to have a virtual function that corresponds to the function of real (not virtual) X-ray sources and real X-ray detectors. This virtual X-ray setup is configured to resemble, simulate or mimic in silico (virtually) the functions of a real X-ray setup comprising, in one embodiment, an X-ray source and an X-ray detector. Accordingly, a virtual X-ray setup according to the invention is configured to simulate or mimic a real X-ray process in silico (virtually). A skilled person is aware of the functions of real X-ray sources and X-ray detectors and their setup producing an outcome similar to a real X-ray image.

In the context of the present invention **"severed"** or **"pathological"** relate to symptomatic anatomy (bone) that might be injured, deformed, maligned, unhealthy, fractured, hunched, comprising an excrescence, traumatized and/or diseased.

A severed or pathological body part may comprise at least one bone, at least one organ, at least one joint, at least one soft tissue, at least one connective tissue, at least one tendon, at least one ligament, at least one cartilage region, at least one meniscus, or any combination thereof.

In one embodiment the severed body part comprises a fracture, deformation or injury selected from the group comprising at least one open fracture, closed fracture, partial fracture, complete fracture, stable fracture, displaced fracture, transverse fracture, spiral fracture, greenstick fracture, stress fracture, compression fracture, oblique fracture, impacted fracture, segmental fracture, comminuted fracture, avulsion fracture, or any other fracture, a deformation, a hunch, an excrescence or any combination thereof.

In one embodiment of the invention the severed or pathological body part or the individual comprising said severed body part is in the need of cosmetical, medical, pharmaceutical, orthopedic, and/or surgical treatment.

Herein the term **"graphical user interface"** or **"GUI""** relates to a form of user interface that allows a user to interact with electronic devices through a graphical representation, graphical icons and/or audio-visual or haptic instead of text-based user interfaces, typed command labels or text navigation. Herein a GUI may be a system of interactive visual components for computer software that displays objects that convey information and represent actions that can be taken by as user. Herein a **"graphical user interface system"** relates to a system configured to facilitate the display of a graphic, a picture, an illustration or the 2-D X-ray images or pseudo-CT, or 3-D representations according to the present invention and/or the interaction of the user with a device or computer connected to the graphical user interface system. Accordingly, herein a graphical user interface system may be, without being limited to, a display, a touch display, a haptic force-feedback device, a display connected to a keypad and/or a computer and/or another device, a computer, a smart phone, a tablet, a server, a medical device, an X-ray device, a surgical device, a Virtual Reality (VR) device, an Augmented Reality (AR) device, or any computer system or device.

In the context of the present invention the term **"sampling"** means transforming the representation of the essentially same data object in various ways, thereby modifying the information content in a controlled way. The term comprises the meaning of projecting, discretizing, digitizing, calculating, estimating, matching, generating, without being limited thereto.

In the context of the present invention the term **"adjusting"** comprises the meaning of adjusting, fitting, changing, amending, modifying or adapting, for example a feature or parameter to control or influence the match of a corresponding feature.

In the context of the present invention the term **"match"** comprises the meaning of matching, adapting, modifying, fitting, or amending a parameter to resemble or match another parameter, such as a feature.

The term **"DICOM"** stands herein for digital imaging and communications in medicine. DICOM is an open standard for storing and exchanging medical image data. Most manufacturers of medical imaging systems such as X-ray machines, magnetic resonance tomographs or computer tomographs use the DICOM standard for image transmission and data processing in their products.

The term **"at least one"** may herein refer to at least one, more than one, at least two at least three, at least four, at least five, at least six, at least seven, at least eight, at least nine, at least ten, at least fifteen, at least twenty, 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, 100, 500, 1000, 10.000.

Particular examples of the presently disclosed methods, devices and computer and programs are as follows.

### Preoperative implant configuration

For joint replacement, bone fracture or bone/joint correction surgery, a suitable implant type and the configuration of the respective implant components (e.g. joint socket and stem, screws, plates) are typically selected from 2-D X-ray images before the procedure. However, this approach often doesn't work perfectly in everyday clinical practice, wherefore for each procedure further configurations and components still have to be kept ready, in case of incorrect prior assessment of the treatment target structures. If a configuration of components does not fit, another configuration is tried. This often involves logistical problems, expenditure of time, an increased health risk for the patient and additional costs.

### Size estimation

Since precise and reliable size estimation cannot be determined from 2-D X-ray images, either several images must be taken from different directions and subsequently compiled or so-called calibration markers are incorporated in the image setup. The exact dimensions of the marker and its distance from the source and/or the detector are recorded and can subsequently be used of determining the size of closely adjacent structures in the 2-D X-ray image. Both approaches can be easily integrated into the method of the present invention and lead to an improved determination of relevant 3-D parameters and thus an improved 3-D representation of the imaged body part.

The precision of the present method in generating 3-D representations from 2-D X-ray images can be further improved, if certain distance measurements and/or empty measurement of the following configurations are carried out and this information is integrated directly into the evaluation parameters (DICOM metadata): a) distance between X-ray source and detector, b) measurement of the distance between X-ray source and the front of the subject, and c) measurement of the distance between the detector and the rear of the subject. Upon integration of this information into the calculation according to the method of the invention the precision and reproducibility of generating properly sized 3-D models or 3-D representations of body parts from 2-D X-ray images can be significantly improved.

### Dose reduction in imaging

If several recordings have to be made from different directions, the present method facilitates the determination of the ideal directions for additional information gain. This eliminates unnecessary exposures and the reduction of the total radiation dose. This applies, in particular, to so-called C-arm X-ray imaging, in which the source and detector are rotated around an isocenter in which the subject is located. For a 3-D reconstruction, at least 180° must be traversed in small angular steps. The method according to the invention facilitates the significant reduction of the required number of exposures and thus the reduction of the radiation dose for the imaged subject by iteratively proposing the next imaging direction that leads to a most significant gain in information.

### Intraoperative navigation (implementation control)

In bone reconstruction or joint replacement surgery planned implant configuration components (e.g. joint socket and stem, screws, plates) should be implemented as precisely as possible in order to restore the desired function. The exact implementation of a planned joint replacement, for example, is not trivial. In particular, in hip-replacement surgery the tilting of the pelvis while lying down and the lack of information about the alignment of the joint socket in relation to the femur during planning often lead to unexpected complications during surgery and after. If intraoperative imaging (e.g. C-arm) is used, one or a few images can be taken during the procedure to check whether the current implant position corresponds to the planned arrangement. In this setup the method and/or the device according to the present invention can be used to aid positioning of implants, screws and/or plates and on further surgical measures or adjustments. Trajectories for bone screws can be specified and controlled more easily, since the X-ray source can be aligned appropriately for the respective intraoperative situation using the knowledge from the 3-D representation generated according to the method of the invention.

### Functional analysis - fluoroscopy (dynamic radiography)

The method according to the invention can be used for the dynamic analysis of joint structures in motion (flexion / extension). CT imaging is not suitable in cases where three-dimensional functional analysis is required, as extensive movements cannot be carried out in a tomograph and tomographic imaging is not fast enough to completely record the movement in place and time. Therefore, X-ray fluoroscopy is used as an alternative, which allows for the provision of X-ray movies for structures in motion. Unfortunately, fluoroscopy is only possible in planar 2-D projection. The method according to the present invention facilitates the morphing of a 3-D model or of previously recorded tomography data to the 2-D fluoroscopy data set, thereby enabling the provision of a sequence of 3-D representations of a moving subject or body part according to the given 2-D X-ray image sequence.

### Normal shape estimation (desired/native/healthy vs. actual state comparison)

Another technological improvement the method according to the present invention achieves is the possibility of a 3-D comparison of a desired or native or healthy vs. an actual pathological status of a body part using 2-D X-ray images. In order to create a representation of the desired physiological state of a body part an A-SSIM derived from a physiological population is matched with image information from the actual 2-D X-ray image. Subsequently it is evaluated how much the information of the actual image deviates from the desired or native or healthy image information. This embodiment of the present method can be useful, for example, to project an anatomical 3-D model that was created based on information from a healthy population to a 2-D X-ray image of a pathological structure of a body part followed by quantification of the deviations or mismatch between both representations in 3-D. In this way, a suggestion for a correction of the pathological structure of the body part or the arrangement of severed or pathological body part, e.g. a broken or malformed bone, can be made.

### FIGURES

The invention is further described by the following figures. These are not intended to limit the scope of the invention but represent preferred embodiments of aspects of the invention provided for greater illustration of the invention described herein.

### Detailed description of the figures:

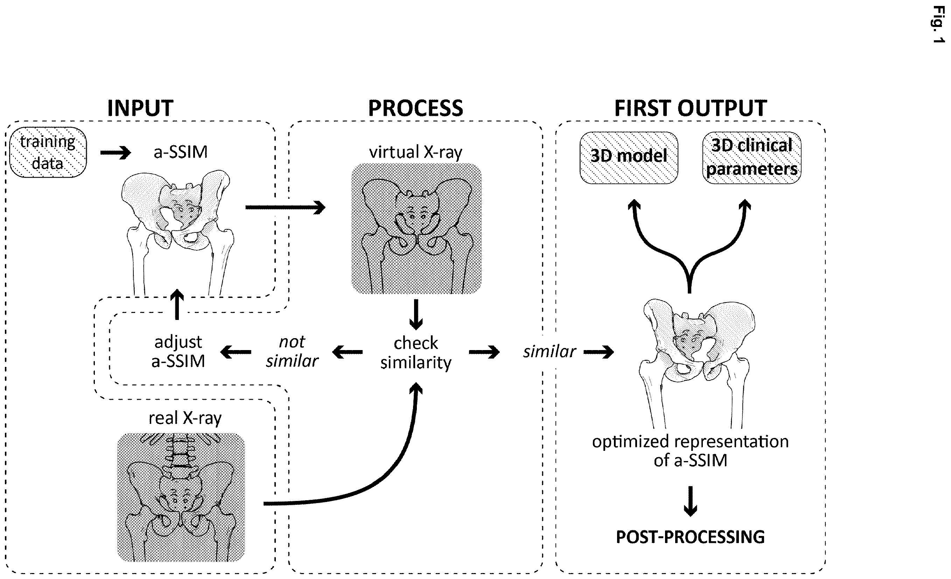

**Figure 1****:** Intensity-based registration of a 3-D model (a-SSIM) to an 2-D X-ray. Training data is used to create an a-SSIM. The candidates for a potential match are selected from the statistical model. To evaluate the fit, virtual X-ray images are projected from the candidates and then compared to the original radiographs by means of 2-D similarity measures. This process iterates until the anatomical representation that best fits the intensity information in the clinical (real X-ray) radiograph is found. This process results in an optimized representation of an a-SSIM. It can be used as a 3D model and for the computation of 3D clinical parameters.

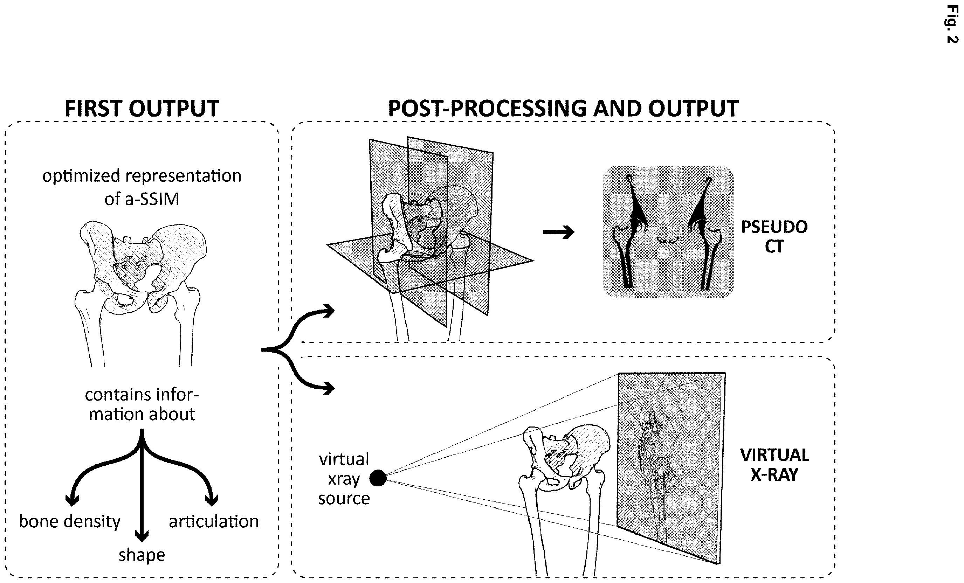

**Figure 2****:** Particular embodiments of the method according to the invention. The illustration depicts the process of generating a virtual/ pseudo CT data set and virtual X-rays. The optimized representation of the a-SSIM not only contains information about the shape but also about the bone density and the articulation. This information is used for the generation of a virtual/ pseudo CT data set and/or a virtual X-ray.

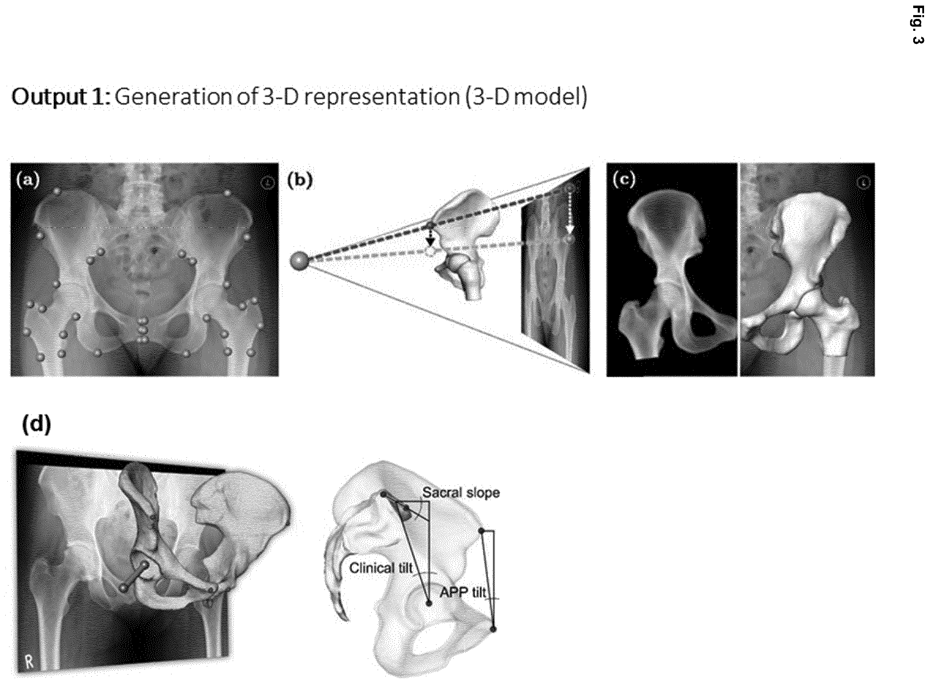

**Figure 3****:** Example of automated reconstruction of the pelvis from a single radiograph. Here 32 landmarks are detected in the AP radiograph (a), corresponding 3-D model points (blue) are morphed to back-projected landmarks for initialization (b), and virtual and reference 2-D radiographs are compared to match the anatomical shape (c). Anatomical parameters of the hip. Four points at the iliac spines and pubic tubercles define the anterior pelvic plane (APP), another set of landmarks around the acetabular rim characterize the orientation of the acetabulum (in blue). The sacral slope is obtained by connecting two implicit landmarks and projecting them into sagittal plane. Clinical tilt is the oriented angle between the line connecting sacral midpoint and the midpoint of the hip axis. The APP tilt relates the orientation of the APP to the X-ray plane (d).

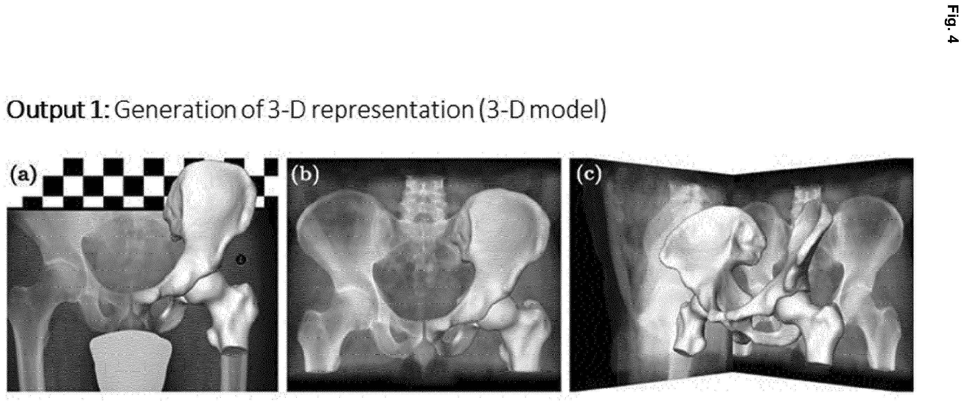

**Figure 4****:** Reconstructed surfaces via the automated method and reference radiographs. (a) clinical radiograph overlapped with reconstruction (hemipelvis); DRR and reconstruction of the same patient in (b) single-view and (c) dual-view setup.

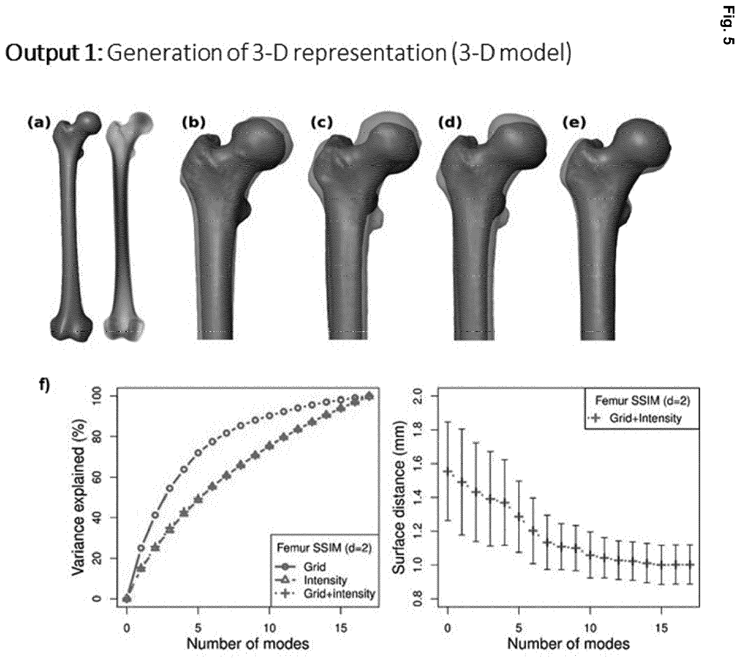

**Figure 5****:** Shape and intensity model of the right femur. The mean model (a) containing shape and intensity information is deformed using the first four modes of variation (b-e). Depicted here is the variation in shape, with opaque surfaces representing the deformation in negative direction and transparent surfaces in positive direction of the respective mode. (f) Compactness and generalization ability of the femur SSIMs with intensity functions of degree d = 2. The generalization ability of the model is given in terms of average surface distances and standard deviations (black bars) when fitting the combined shape/intensity to unseen datasets using the n most significant modes of variation.

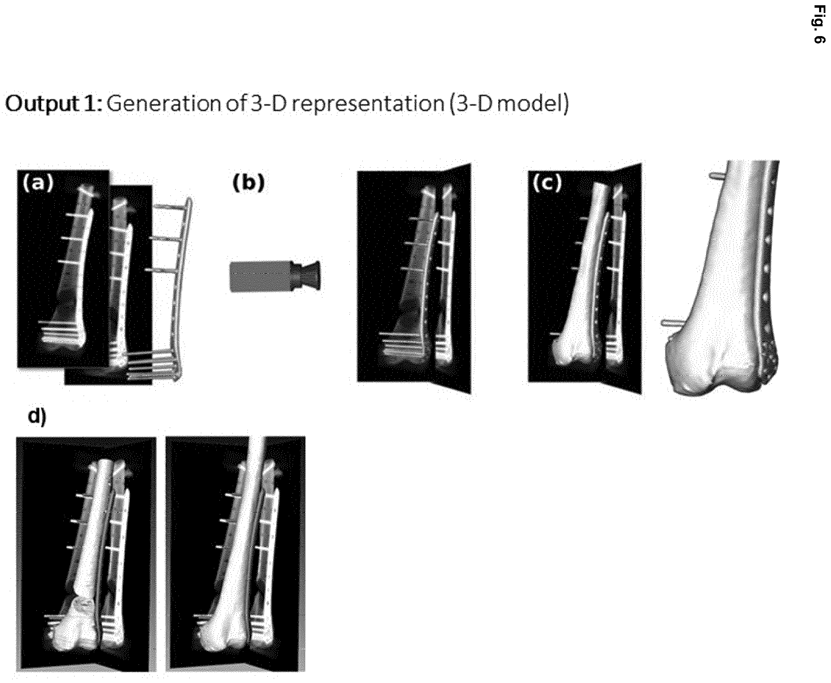

**Figure 6****:** Overview of the reconstruction pipeline for deriving the plate and femur. Two radiographs and the implant geometry are given as an input (a). The implant geometry is registered to the X-rays in order to derive the X-ray calibration and implant pose (b). A SSIM of the femur is then fitted to both radiographs simultaneously, resulting in the 3-D shape of the patient-specific femur in relative pose to the implant as depicted in the reference images. Comparison between ground-truth and 3-D reconstruction from 2-D radiographs (Ehlke et al., 2015). Ground-truth shape of the femur and implant from CT (left) of case 1-L and the 3D-reconstructed femoral shape and implant pose based on reference DRRs (right). The SSIM extrapolates over the fracture gap depicted in the X-rays (d).

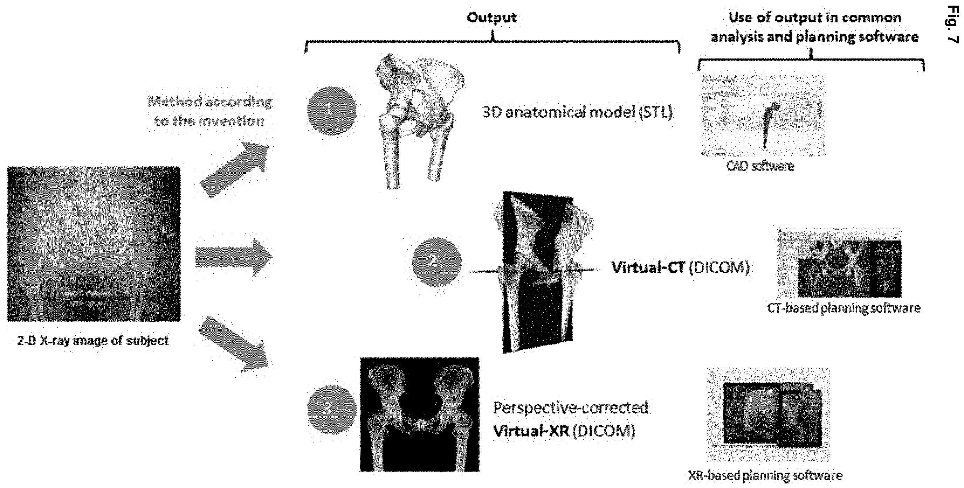

**Figure 7****:** Particular embodiments of the method according to the invention. The illustration depicts a general overview of three outputs that can be generated with the method according to the invention, based on at least one 2-D X-ray image. The obtained output, such as (1) a 3-D anatomical model representation of the imaged body part, (2) virtual CT data sets and (3) virtual 2-D X-ray images generated either from 3-D models or from virtual CT datasets, can be analyzed, displayed and further processed by common / standard computer programs for diagnostics, treatment planning, as well as prosthesis design.

### EXAMPLES

The invention is further described by the following examples. These are not intended to limit the scope of the invention but represent preferred embodiments of aspects of the invention provided for greater illustration of the invention described herein.

### Example 1

The performance of the initialization and reconstruction stages was evaluated quantitatively by comparing the reconstructed shapes of the hip-joint to ground-truth surfaces from 24 clinical CTs. For 10 CTs, AP radiographs of the same patients were available that showed the hip-joint in various pathological stages of femoral acetabular impingement (FAI). The proximal parts of the ilia and sacrum were not visible in eight of the images. Four patients wore gonadal shields that overlapped with the pelvis in the radiographs (Figure 4a). The other 14 CTs were preselected from the training base of the A-SSIM such that they show the full pelvis, proximal femur and upper parts of the femoral shaft. Digitally reconstructed radiographs (DRRs) were generated from all CTs, mimicking 2-D radiographs in clinical single-view (AP) and dual-view (AP and sagittal) setups with a simulated source-detector distance of one meter (Figures 4b and c).

The goal was to assess the performance of the initialization method based on auto-mated landmark detection, the surface reconstruction errors after intensity-based reconstruction, as well as the extraction of anatomical parameters using vertex correspondences in single-view and dual-view setups. For this purpose, the DRRs and clinical radiographs were subdivided into three groups:
- FAI-XRAY contains the 10 clinical AP radiographs of the FAI cases.
- FAI-DRR contains AP and sagittal DRRs from the corresponding CTs of the FAI cases.
- MODEL-DRR contains AP and sagittal DRRs that were generated from the training base (CTs) of the A-SSIM.

### Landmark detection and initialization

The performance of the proximal femur FALA system was previously evaluated in Lindner et al. (2013). The pelvis FALA system was assessed using three-fold cross-validation experiments based on manually annotated ground-truth. It showed an average point-to-curve error of less than 1.8mm for 99% of all 150 images. Of note is that this performance was achieved using only 100 training images in a dataset with considerable variation due to gender, positioning and anatomical variation. Average re-projection (2-D) error per landmark after initialization of the statistical model was measured as 2.4mm (FAI-XRAY, 10 datasets), 2.2mm (FAI-DRR, 10 datasets) and 2.3mm (MODEL-DRR, 14 datasets), with the first five modes of shape variation optimized in the initialization stage. A qualitative assessment of the results showed no obvious misfits between A-SSIM and X-rays, such as alignment of the wrong anatomy (pelvis fitted to the femur) or inverted orientations of the model (posterior-anterior pose in an AP setup, toe-to-head pose in head-to-toe setup). Quantitative values for the three experimental series are given in the first rows of Table 1, com-paring the surface distances and orientation of the pelvis to the ground-truth from CT.

### Shape reconstruction

The reconstructed anatomical shapes were assessed by measuring the Euclidean surface distance to ground-truth shapes from CT. For this purpose, 2-D/3-D reconstructions were performed from the given clinical radiographs and DRRs using the 20 most significant modes of variation of the A-SSIM, which together explained 67% of the shape and intensity variation in the training population. The experiments were then repeated using the full shape and intensity parameter space of the model.

The average point-to-surface distance between surfaces of the A-SSIM and ground-truth was recorded after landmark-based initialization as well as after intensity-based registration (full reconstruction). Before evaluating surface distances, the ilia and femurs of the model were aligned rigidly to the ground-truth in order eliminate the effects of global transformation and articulation on the measure. The reconstruction error in anatomical pose was assessed separately by measuring the differences in angle of the APP normal between ground-truth and reconstruction result.

Table 1, first column, lists the results from the 10 clinical radiographs (FAI-XRAY) in terms of mean distance and deviation over all experiments. Since the patient-specific pose in the original X-ray setup was unknown, the APP could not be compared to ground-truth directly and is hence marked as "n/a" in the table. It was, however, possible to estimate the true, patient-specific orientation by rigidly aligning the ground-truth shapes to the clinical X-ray images. Refer to Table 3 for a comparison of the reconstructed pelvic tilt to the estimated values from ground-truth.

The results from the DRR-based experiments (FAI-DRR and MODEL-DRR) are given in Table 1, columns two and three. The MODEL-DRR experiments were per-formed both with the full model (full) and in a leave-out fashion, where the training set depicted in the respective DRRs was removed from the model before reconstruction. The run-time per reconstruction was less than five minutes for single-view and less than ten minutes for dual-view setups.

The optional refinement stage was further applied on the FAI experiment series in order to study the impact of less restrictive geometric priors in 2-D/3-D reconstruction. In single-view scenarios, the reconstruction accuracy with respect to the ilia was reduced to 2.0mm (FAI-XRAY) and 1.9mm (FAI-DRR) when using refinement. The reconstruction accuracy was, however, slightly improved in dual-view experiments (1.5mm).

**Table 1: Reconstructed surfaces from clinical radiographs (FAI-XRAY) and DRRs (FAI-DRR, MODEL-DRR) compared to ground-truth. The error is given in terms of mean Euclidean surface distance, averaged over pairs of ilia and femurs (± standard deviation). The error in reconstructed anatomical pose is expressed as the mean angle between the normals of the APP from the reconstruction and from the ground-truth. Full: Ground-truth instance was contained in the A-SSIM; Leave-out:lnstance was removed from the A-SSIM prior to reconstruction.**

| | **FAI-XRAY** (*n* = 10) | **FAI-DRR** (*n* = 11) | **MODEL-DRR** (*n* = 14) | |
|---|---|---|---|---|
| **Landmark-based initialization** | | | | |
| Ilia (mm) | 2.46 *(*±*0.57)* | 2.47 *(*±*0.59)* | 2.55 *(*±*0.40)* | |
| Femurs (mm) | 2.06 *(*±*0.59)* | 1.79 *(*±*0.29)* | 1.59 *(*±*0.54)* | |
| APP-angle (°) | n/a | 4.94 *(*±*2.63)* | 5.71 *(*±*3.53)* | |
| **Single-view reconstruction (20 modes)** | | | **leave-out** | **full** |
| Ilia (mm) | 1.86 *(*±*0.39)* | 1.77 *(*±*0.38)* | 1.97 *(*±*0.28)* | 0.97 *(*±*0.38)* |
| Femurs (mm) | 1.35 *(*±*0.44)* | 1.33 *(*±*0.38)* | 1.31 *(*±*0.30)* | 0.59 *(*±*0.38)* |
| APP-angle (°) | n/a | 3.19 *(*±*2.19)* | 3.71 *(*±*1.96)* | 1.97 *(*±*1.60)* |
| **Single-view reconstruction (45 modes)** | | | **leave-out** | **full** |
| Ilia (mm) | 1.91 *(*±*0.35)* | 1.73 *(*±*0.29)* | 2.07 *(*±*0.39)* | 0.63 *(*±*0.20)* |
| Femurs (mm) | 1.28 *(*±*0.38)* | 1.30 *(*±*0.39)* | 1.24 *(*±*0.33)* | 0.33 *(*±*0.08)* |
| APP-angle (°) | n/a | 2.35 *(*±*1.24)* | 4.01 *(*±*2.50)* | 1.48 *(*±*0.70)* |
| **Dual-view reconstruction (45 modes)** | | | **leave-out** | **full** |
| Ilia (mm) | n/a | 1.59 *(*±*0.35)* | 1.77 *(*±*0.34)* | 0.53 *(*±*0.19)* |
| Femurs (mm) | n/a | 1.19 *(*±*0.34)* | 1.21 *(*±*0.28)* | 0.35 *(*±*0.11)* |
| APP-angle (°) | n/a | 2.34 *(*±*1.66)* | 2.30 *(*±*1.35)* | 0.56 *(*±*0.62)* |

**Table 2: Reconstructed intensities from the MODEL-DRR experiments compared to ground-truth. The results are given in terms of mean absolute error in Hounsfield units between intensities from ground-truth model instances and the intensities sampled from the reconstruction outcome, averaged over pairs of ilia and femurs (± standard deviation). Full: Ground-truth instance was contained in the A-SSIM; Leave-out: Instance was removed from the A-SSIM prior to reconstruction.**

| **MODEL-DRR Leave-out MODEL-DRR Full** | | |
|---|---|---|
| **Single-view reconstruction (20 modes)** | | |
| Ilia | 140.12 *(*±*26.69)* | 67.77 *(*±*25.83)* |
| Femurs | 119.23 *(*±*16.63)* | 55.17 *(*±*21.19)* |
| **Single-view reconstruction (45 modes)** | | |
| Ilia | 146.97 *(*±*18.82)* | 40.52 *(*±*16.48)* |
| Femurs | 116.34 *(*±*12.00)* | 30.68 *(*±*12.12)* |
| **Dual-view reconstruction (45 modes)** | | |
| Ilia | 138.34 *(*±*14.84)* | 46.10 *(*±*24.89)* |
| Femurs | 113.51 *(*±*14.21)* | 37.94 *(*±*22.91)* |

**Table 3: Anatomical parameters from clinical radiographs and DRRs compared to ground-truth (± standard deviation) in terms of absolute differences in angle (degrees). APP tilt represents the inclination angle of the anterior pelvic plane. Clinical tilt refers to the angle between midpoint of the sacral plateau and hip axis. The sacral slope was computed w.r.t. the X-ray plane in AP view. Acetabular anteversion and inclination parameters are given relative to the APP.**

| | **FAI-XRAY** (*n* = 10) | **FAI-DRR** (*n* = 11) | **MODEL-DRR** (*n* = 14) | |
|---|---|---|---|---|
| **Single-view reconstruction (20 modes)** | | | **leave-out** | **full** |
| APP tilt | 3.5 *(*±*3.4)* | 3.0 *(*±*2.4)* | 3.7 *(*±*3.0)* | 2.1 *(*±*1.7)* |
| Clinical tilt | 2.9 *(*±*3.0)* | 3.7 *(*±*3.4)* | 4.1 *(*±*3.1)* | 2.1 *(*±*1.6)* |
| Sacral slope | 7.2 *(*±*3.8)* | 7.6 *(*±*3.7)* | 10.1 *(*±*7.2)* | 1.8 *(*±*1.6)* |
| Acet. version | 3.9 *(*±*2.9)* | 3.3 *(*±*2.4)* | 4.1 *(*±*3.1)* | 2.0 *(*±*1.2)* |
| Acet, inclination | 3.4 *(*±*3.3)* | 3.6 *(*±*2.8)* | 3.6 *(*±*2.0)* | 1.7 *(*±*1.8)* |
| **Single-view reconstruction (45 modes)** | | | **leave-out** | **full** |
| APP tilt | 3.3 *(*±*2.9)* | 2.3 *(*±*0.8)* | 4.5 *(*±*3.5)* | 1.6 *(*±*0.8)* |
| Clinical tilt | 3.8 *(*±*4.5)* | 2.7 (±2.1) | 3.7 *(*±*2.5)* | 1.4 (±1.0) |
| Sacral slope | 7.9 *(*±*4.5)* | 5.4 *(*±*3.8)* | 8.7 *(*±*5.4)* | 1.3 *(*±*1.1)* |
| Acet. version | 3.2 *(*±*2.3)* | 3.2 *(*±*2.3)* | 4.5 *(*±*2.8)* | 1.5 *(*±*1.0)* |
| Acet. inclination | 4.2 *(*±*3.1)* | 3.9 *(*±*2.8)* | 3.6 *(*±*2.0)* | 1.0 *(*±*0.5)* |
| **Dual-view reconstruction (45 modes)** | | | **leave-out** | **full** |
| APP tilt | n/a | 1.5 *(*±*1.9)* | 3.0 *(*±*2.0)* | 0.6 *(*±*0.6)* |
| Clinical tilt | n/a | 2.9 *(*±*2.2)* | 2.6 *(*±*1.7)* | 1.3 *(*±*1.2)* |
| Sacral slope | n/a | 5.9 *(*±*5.2)* | 8.9 *(*±*5.0)* | 2.0 *(*±*1.7)* |
| Acet. version | n/a | 2.7 *(*±*1.2)* | 3.3 *(*±*2.8)* | 0.8 *(*±*0.7)* |
| Acet, inclination | n/a | 3.4 *(*±*2.8)* | 3.0 *(*±*2.3)* | 0.8 *(*±*0.9)* |
| | | | | |

### Intensity reconstruction

The intensities reconstructed in the MODEL-DRR experiments were compared to the ground-truth intensities from the corresponding training sets of the A-SSIM. To evaluate intensity information exclusively, shape and transformations of the reconstruction outcome were normalized following the approach of sampling Bernstein coefficients from CT. The Bernstein coefficients from the ground-truth instances, which originated from the CTs that the DRRs were generated from, were mapped to the mean tetrahedral grid. The 2-D/3Dreconstructed coefficients from the DRR-based experiments were transferred onto the same tetrahedral grid, making use of the vertex correspondence between model instances. Both polynomial distributions (reconstructed and ground-truth) were then sampled as Hounsfield units onto CT-like voxel grids of resolution 0.5 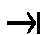 0.5 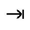 0.5mm and evaluated against each other, ignoring surrounding voxels that were located outside the mean tetrahedral grid. The results are given in Table 2 for single-view and dual-view experiments.

### Reconstruction of anatomical parameters

The anatomical parameters of the imaged body part were derived from the reconstructed shapes and then evaluated with respect to the patient-specific pose in the given X-ray setups. To establish the ground-truth pose from clinical radiographs (FAIXRAY), the CTs of the patients were fitted rigidly to the given X-ray images by means of 2-D/3-D registration. The outcome was then confirmed qualitatively by an expert observer. The ground-truth pose in the DRR-based experiments (FAI-DRR, MODELDRR) was given at the time of DRR generation by the orientation of the CT datasets relative to the camera setup (extrinsic camera parameters). Table 3 compares the anatomical parameters from the shape reconstruction experiments to ground-truth from CT. The APP, clinical tilt, sacral slope and acetabular anteversion/inclination were computed from anatomical 3-D landmarks on the reconstructed shapes and in the CTs. The landmarks on the reconstructed shapes were extracted automatically using vertex identifiers and the hip-joint center of the A-SSIM. Corresponding ground-truth landmarks were defined manually on the CTs and then transformed in the X-ray setups using the known pose of the patients.

### Example 2

### SSIMs of the femur

Following the method according to the invention, SSIMs of the whole femur were generated based on 18 preoperative CT scans of mixed male and female patients that were treated for osteoarthritis of the knee. The images showed either the right or left side of the lower limb together with the hip and knee joints at a resolution below 0.5 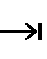 0.5 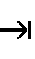 0.9mm per voxel in all datasets. The CTs were mirrored along the transverse axis to represent opposite sides, resulting in 18 training sets for the left and the right femur. Each set was segmented using the automated approach described in Bindernagel et al. (2011) and segmentations later refined manually by an expert observer.

An existing SSM of the femur, originally created from 95 MRIs in previous experiments, was utilized to establish vertex correspondence with respect to outer points. The SSM was fitted to 3D label field representations of the segmented CTs, mimicking the iterative approach proposed in Bindernagel et al. (2011); Kainmueller et al. (2009). Training shapes were then extracted by projecting the vertices of the matched SSMs along their normal direction to the segmented contours. The vertices and triangulation of the original SSM were thereby transferred to the segmentations from CT. In order to establish correspondence on inner points, a template mesh of the mean shape, consisting of 140k tetrahedra, was morphed to the training surfaces via mean value coordinates deformation (Floater, 2003). The intensities were incorporated into the model by sampling Bernstein intensity functions of degree d = 2 from the CTs onto the meshes. A PCA was then computed on the combined shape and intensity information as well as for shape and intensity independently. The combined shape and intensity model of the right femur is depicted in Figure 5. The most significant modes of variation express the scale and orientation of the femoral neck and trochanter, cortical thickness, as well as the length and thickness of the femoral shaft. High-frequency variations of the anatomical shape due to pathologies in the joint regions are captured by the least significant modes of the model. The compactness and generalization ability of the femur SSIM were evaluated for Bernstein intensity functions of second order. The compactness was analyzed based on the eigenvalues associated with a PCA on the tetrahedral meshes in the model, as well as for the combined PCA on shape and intensity information. A leave-one-out cross validation was performed on the SSIM to measure the generalization ability in terms of average surface distance to unseen training data across the most significant modes of variation. The results of the analysis on compactness and generalization ability are depicted in Figure 5f.

### Evaluation based on DRRs

The evaluation utilizes CT datasets that have originally been acquired for an assessment of interfragmentary lag screw fixation in locking plate constructs (Märdian et al., 2015). The CTs depict four pairs of cadaveric distal femoral bones and two individual femurs, with a fracture gap at the distal shaft. Surrounding tissue is not present in the datasets. A standard 9-hole 4.5/5.0 LISS DF2 plate fixates the fracture using seven locking screws distally and three screws proximally. Based on the CT scans, digitally reconstructed radiographs (DRRs) were generated in AP and mediolateral view in order to mimic clinical X-ray images taken at a source detector distance of one meter. The global X-ray setup was then derived as proposed using a CAD model of the implant. Afterwards, the 3-D shape of the femur was reconstructed in the global X-ray setup. The cadaveric bones were not contained in the training base of the statistical prior. A surface model of the intact bone was registered to the CT data, which then acted as ground-truth for evaluation. To assess the distance between implant and bone at consistent point locations, an ideal plate geometry was registered rigidly to each reconstructed plate as well as to the plates depicted in the CTs. The surface distance between the reconstructed bone surface from the statistical model and the reconstructed plate as well as the surface distance between the intact bone and the ground-truth plate were then compared to each other. In addition, the surface error between intact femur and reconstructed femur was assessed after rigid registration of the reconstructed femoral shape to the ground-truth.

**Table 4: Error in plate-to-bone distance in mm between reconstructed plate and femur against ground-truth per case. Listed are the root-mean-square error (RMSE), mean error as well as the maximum error over all plate nodes including proximal, distal and mid-section of the osteosynthesis. L and R denote left and right femur respectively, with results from both sides listed when available. Cases 5-L and 6-R were treated as outliers, since the implant plate penetrated the femur.**

| | **1-L/R** | **2-L/R** | **3-L/R** | **4-L** | **5-L/R** | **6-R** |
|---|---|---|---|---|---|---|
| RMSE [mm] | 1.5 / 1.2 | 1.3 / 1.5 | 1.7 / 1.2 | 1.36 | 1.9 / 1.7 | 2.6 |
| Mean [mm] | 0.1 / 0.8 | 0.3 / 0.8 | 0.6 / 0.2 | 0.2 | 1.5 / 1.1 | 1.5 |
| Max [mm] | 4.7 / 2.4 | 4.0 / 4.3 | 5.1 / 4.1 | 4.9 | 3.7 / 5.2 | 6.0 |

Figure 6 exemplary shows the osteosynthesis from CT compared to the reconstructed femur and implant for case 1-L. The mean error in plate-to-bone surface distance over the whole plate is given for each case individually in Table 4. Root-mean-square-error (RMSE) was at most 2.6mm for all tested cases, with an absolute mean deviation smaller than 1.5mm. The surface of the femur penetrated the implant in reconstructions of cases 5-L and 6-R, thus representing anatomically implausible osteosynthesis constructs. Excluding these outliers yields an overall RMSE of at most 1.7mm and a maximum mean error of 1.1mm. Further restriction of nodes to the midsection of the plate results in RMSE less than 1.4mm, with the span ranging from -2.4mm to +2.9mm. Compared to the intact bone surface, the femoral surface is reconstructed with a mean error of at most 1.5mm, excluding cases 5-L and 6-R. The two outliers exhibit a mean error of 1.8mm and 2.0mm respectively.

### References

M. Ehlke, H. Ramm, H. Lamecker, H. Hege and S. Zachow, "Fast Generation of Virtual X-ray Images for Reconstruction of 3D Anatomy," in IEEE Transactions on Visualization and Computer Graphics, vol. 19, no. 12, pp. 2673-2682, Dec. 2013, doi: 10.1109/TVCG.2013.159.

S.Z. Aydin, Kasapoglu G.E., E. Kurum, S. Akar, H.E. Mungan, F. Alibaz-Oner, R.G. Lambert, P. Atagunduz, H. Marzo Ortega, D. McGonagle, and W.P. Maksymowych. Limited reliability of radiographic assessment of spinal progression in ankylosing spondylitis. Rheumatology, 56(12):2162-2169, 2017.

M. Ehlke, M. Heyland, S. Märdian, G.N. Duda, and S. Zachow. 3D assessment of osteosynthesis based on 2D radiographs. In Jahrestagung der Deutschen Gesellschaft fürComputer- und Roboterassistierte Chirurgie (CURAC),2015.ZIB-Report15-4.

C. Lindner, S. Thiagarajah, J.M. Wilkinson, The arcOGEN Consortium, G.A. Wallis,and T.F. Cootes. Fully automatic segmentation of the proximal femur using random forest regression voting. IEEE Transactions on Medical Imaging,32(8):1462-1472,2013.

J.C. Clohisy, J.C. Carlisle, R. Trousdale, Y.J. Kim, P.E. Beaule, P. Morgan, K. Steger-May, P.L. Schoenecker, and M. Millis. Radiographic evaluation of the hip has limited reliability. Clinical Orthopaedics and Related Research, 467(3):666-675, 2009.

G. Zheng. Statistically deformable 2D/3D registration for accurate determination of post-operative cup orientation from single standard X-ray radiograph. In Medical Image Computing and Computer-Assisted Intervention (MICCAI), pages 820-827, 2009.

S. Schumann, Y. Sato, Y. Nakanishi, F. Yokota, M. Takao, N. Sugano, and G. Zheng. Cup implant planning based on 2-D/3-D radiographic pelvis reconstruction - First clinical results. IEEE Transactions on Biomedical Engineering, 62(11):2665-2673, 2015.

N. Baka, B.L. Kaptein, M. de Bruijne, T. van Walsum, J.E. Giphart, W.J. Niessen, and B.P. Lelieveldt. 2D-3D shape reconstruction of the distal femur from stereo X-ray imaging using statistical shape models. Medical Image Analysis, 15(6):840-850, 2011.

N. Baka, B.L. Kaptein, J.E. Giphart, M. Staring, M. de Bruijne, B.P. Lelieveldt, and E. Valstar. Evaluation of automated statistical shape model based knee kinematics from biplane fluoroscopy. Journal of Biomechanics, 47(1):122-129, 2014.

J. Dworzak, H. Lamecker, J. von Berg, T. Klinder, C. Lorenz, D. Kainmüller, H. Seim, H.-C. Hege, and S. Zachow. 3D reconstruction of the human rib cage from 2D projection images using a statistical shape model. International Journal of Computer Assisted Radiology and Surgery, 5(2):111-124, 2010.

H. Lamecker, T.H. Wenckebach, and H.-C. Hege. Atlas-based 3D-shape reconstruction from X-ray images. In International Conference on Pattern Recognition (ICPR), volume 1, pages 371-374, 2006.

G. Zheng. Statistically deformable 2D/3D registration for accurate determination of post-operative cup orientation from single standard X-ray radiograph. In Medical Image Computing and Computer-Assisted Intervention (MICCAI), pages 820-827, 2009.

T. Whitmarsh, L. Humbert, M. De Craene, L.M. Del Rio Barquero, and A.F. Frangi. econstructing the 3D shape and bone mineral density distribution of the proximal femur from dual-energy X-ray absorptiometry. IEEE Transactions on Medical Imaging, 30(12):2101-2114, 2011.

V. Karade and B. Ravi. 3D femur model reconstruction from biplane X-ray images: A novel method based on Laplacian surface deformation. International Journal of Computer Assisted Radiology and Surgery, 10(4):473-485, 2015.

J. Yao. A statistical bone density atlas and deformable medical image registration. PhD thesis, The Johns Hopkins University, 2002.

J. Georgii and R. Westermann. A generic and scalable pipeline for GPU tetrahedral grid rendering. IEEE Transactions on Visualization and Computer Graphics, 12(5): 1345-1352, 2006.

M. Weiler, M. Kraus, M. Merz, and T. Ertl. Hardware-based view-independent cell projection. IEEE Transactions on Visualization and Computer Graphics, 9(2):163-175, 2003.

O. Sadowsky, J.D. Cohen, and R.H. Taylor. Rendering tetrahedral meshes with higherorder attenuation functions for digital radiograph reconstruction. In IEEE Visualization, pages 303-310, 2005.

O. Sadowsky, J.D. Cohen, and R.H. Taylor. Projected tetrahedra revisited: A barycentric formulation applied to digital radiograph reconstruction using higher-order attenuation functions. IEEE Transactions on Visualization and Computer Graphics, 12(4): 461-473, 2006.

M. Bindernagel, D. Kainmüller, H. Seim, H. Lamecker, S. Zachow, and H.-C. Hege. An articulated statistical shape model of the human knee. In Bildverarbeitung für die Medizin (BVM)), pages 59-63, 2011.

D. Kainmueller, H. Lamecker, S. Zachow, and H.-C. Hege. An articulated statistical shape model for accurate hip joint segmentation. In Annual International Conference of the IEEE Engineering in Medicine and Biology Society, pages 6345-6351, 2009.

M.S. Floater. Mean Value Coordinates. Computer Aided Geometric Design, 20(1):19-27, 2003.

S. Märdian, W. Schmölz, K.-D. Schaser, G.N. Duda, and M. Heyland. Interfragmentary lag screw fixation in locking plate constructs increases stiffness in simple fracture patterns. Clinical Biomechanics, 30(8):814-819, 2015.

## Claims

1. **Method** for obtaining a three-dimensional (3-D) representation of a body part of a subject from at least one two-dimensional (2-D) X-ray image of the body part comprising:
a. providing at least one 2-D X-ray image of a body part of a subject,
b. providing a continuous parametric 3-D model of the body part corresponding to the imaged body part of the subject,
c. adjusting at least one feature of the continuous parametric 3-D model to match the corresponding feature of the at least one 2-D X-ray image of the body part of the subject, wherein the at least one feature is (preferably) selected from the group comprising an anatomical landmark, contour, shape, image intensity, structure and silhouette of the at least one 2-D X-ray image of the body part of the subject, thereby generating a 3-D representation of the imaged body part of the subject from the continuous parametric 3-D model,
d. generating at least one virtual 2-D X-ray image from the 3-D representation of the imaged body part of the subject generated in step c.,
e. determining the difference between the at least one virtual 2-D X-ray image and the at least one 2-D X-ray image of the body part of the subject with respect to the at least one feature,
f. further adjusting the least one feature of the continuous parametric 3-D model to decrease the difference to the corresponding feature of the at least one 2-D X-ray image of the body part of the subject, thereby generating an improved 3-D representation of the imaged body part of the subject from the continuous parametric 3-D model,
g. repeating steps d. to f. until the difference between the at least one virtual 2-D X-ray image generated from the improved 3-D representation and the at least one 2-D X-ray image of the body part of the subject is not further decreased, thereby obtaining a final (or optimized) 3-D representation of the body part of the subject.

2. The method according to claim 1, wherein at least one X-ray marker preferably of defined dimension is present in the 2-D X-ray image and wherein the at least one X-ray marker is used to determine at least one feature selected from the group comprising an anatomical landmark, the distance between the imaged body part and the X-ray source and/or the detector, the size and/or orientation of the imaged body part.

3. The method according to any one of the preceding claims, further comprising **h.** generating a virtual computed tomography (CT) data set from the final 3-D representation of step **g.**

4. The method according to claim 3,
wherein the continuous parametric 3-D model comprises data on variations in shape and image intensity learned from CT-data sets the continuous parametric 3-D model is derived from,
wherein the at least one adjusted feature comprises image intensity, and
wherein step h. comprises sampling the intensity data of the final 3-D representation to a grid, thereby modelling the intensity data in a volume inside and/or outside of the final 3-D representation.

5. The method according to claim 3 or 4, further comprising **i.** generating at least one virtual 2-D X-ray image from the virtual CT data set generated in step **h.**

6. The method according to claim 5, wherein step i. comprises:
- defining a virtual X-ray setup comprising at least a source and a detector,
- virtually arranging the virtual CT data set between the source and the detector of the virtual X-ray setup,
- simulating the X-ray process within the virtual X-ray setup to create at least one virtual 2-D X-ray from the virtual CT data set.

7. The method according to any one of the preceding claims, wherein the imaged body part is a severed body part, and wherein the continuous parametric 3-D model and/or the (final) 3-D representation are a healthy or intact representation of the severed body part.

8. The method according to claim 1 or 2, wherein a continuous fluoroscopic (real-time moving) 2-D X-ray image of a moving body part of a subject is provided, and wherein the (final) 3-D representation is a moving 3-D representation of the imaged body part in motion.

9. The method according to any one of the preceding claims, wherein the body part of the subject is selected from the group of one or more organ(s), limb(s), bone(s), joint(s) and/or a portion and/or a combination thereof.

10. **A device or system** comprising means for carrying out the steps of the method according to any one of claims 1 to 9, and preferably a graphical user interface system configured to display the (final) 3-D representation and/or the at least one virtual CT data set, generated according to claim 3 or 4, of the body part of the subject.

11. **A computer-program [product]** for obtaining a three-dimensional (3-D) representation of a body part of a subject from at least one two-dimensional (2-D) X-ray image of the body part of the subject, comprising instructions which, when the program is executed by a computer, cause the computer to execute the steps of the method according to any one of claims 1 to 9 and to display the (final) 3-D representation and/or the at least one virtual CT data set, generated according to claim 3 or 4, of the body part of the subject on a graphical user interface system.

12. **A computer-readable storage medium** having stored thereon the computer program of claim 9.

13. **Use** of a 3-D representation, relating to the shape and appearance of at a body part, and obtained according to the method according to any one of claims 1 to 9, for the construction of a joint prosthesis or an osteosynthesis plate or prosthesis.

14. **Use** of a 3-D representation obtained according to the method of any one of claims 1 to 9, for the planning and/or the evaluation of the outcome of a surgical procedure on the imaged body part.

15. The method according to any one of claims 1-9, wherein the body part of the subject comprises at least one implant, screw, plate, joint prosthesis, osteosynthesis plate and/or any combination thereof.
